# EUROPEAN PATENT APPLICATION

(11) **EP 4 751 652 A1**
(43) Date of publication of application: **03.06.2026**
(21) Application number: 25218773.7
(22) Date of filing: 26.11.2025
(51) Int. Cl.: A61B 6/04, A61B 6/50

(54) **TWO-DIMENSIONAL MECHANICAL IMAGING SYSTEMS AND METHODS OF USE THEREOF**

(30) Priority: 27.11.2024 US 202418961541
(71) Applicant: Teledyne Dalsa B.V., 5656 AE Eindhoven (NL)
(72) Inventor: SNYDER, Jonathan E, Park City, 84098 (US); LOMAKO, Andriy, Waterloo, N2L 6H5 (CA); NIKIEL, Anna Maria, 5614CD Eindhoven (NL); MILLER, James J, Tavistock, N0B 2RO (CA)
(74) Representative: HGF

(57) **Abstract**

An imaging system for imaging a tissue of a subject. The system comprises a first substrate, a second substrate, and X-ray imaging system, and a two-dimensional pressure sensory array. The second substrate is spaced a first distance from the first substrate. The first substrate and the second substrate are positioned in an imaging field. The first substrate and the second substrate are capable to move relative to one another to during a compression cycle. The X-ray imaging system capable to image the specimen in the imaging field. The two-dimensional pressure sensor array positioned on the first substrate, the second substrate, or both the first substrate and the second substrate, wherein the two-dimensional pressure sensor array is capable to measure a pressure distribution of the specimen during the compression cycle.

## Description

### FIELD

This disclosure relates generally to two-dimensional mechanical imaging systems and methods of use thereof.

### BACKGROUND

Diagnosing breast cancer can involve various examination techniques directed to supporting early and accurate cancer detection, including, for example, X-ray imaging. There are challenges with identifying breast cancer early and efficiently.

### SUMMARY

In one general aspect, the present disclosure is directed to an imaging system for imaging a tissue of a subject. The system comprises a first substrate, a second substrate, and X-ray imaging system, and a two-dimensional pressure sensory array. The second substrate is spaced a first distance from the first substrate. The first substrate and the second substrate are positioned in an imaging field. The first substrate and the second substrate are capable to move relative to one another to during a compression cycle. The X-ray imaging system capable to image the specimen in the imaging field. The two-dimensional pressure sensor array positioned on the first substrate, the second substrate, or both the first substrate and the second substrate, wherein the two-dimensional pressure sensor array is capable to measure a pressure distribution of the specimen during the compression cycle.

Another aspect of the present disclosure is directed to a method for imaging a specimen. The method comprises compressing the specimen positioned between a first substrate and a second substrate. The method further comprises X-ray imaging the specimen. The method further comprises measuring a pressure distribution of the specimen across the first substrate, the second substrate, or both the first substrate and the second substrate.

Although the present disclosure relates to different aspects and embodiments, it is understood that the different aspects and embodiments disclosed herein can be integrated, combined, or used together as a combination system, or in part, as separate components, devices, and systems, as appropriate. Thus, each embodiment disclosed herein can be incorporated in each of the aspects to varying degrees as appropriate for a given implementation.

These and other features of the applicant's teachings are set forth herein.

### BRIEF DESCRIPTION OF THE FIGURES

Unless specified otherwise, the accompanying drawings illustrate aspects of the innovations described herein. Referring to the drawings, wherein like numerals refer to like parts throughout the several views and this specification, several embodiments of presently disclosed principles are illustrated by way of example, and not by way of limitation. The drawings are not intended to be to scale. A more complete understanding of the disclosure may be realized by reference to the accompanying drawings in which:
FIG. 1 is a schematic diagram of a non-limiting embodiment of a two-dimensional mechanical imaging system according to the present disclosure;
FIG. 2A is an illustration of a pressure sensor for use in a two-dimensional pressure sensor array according to the present disclosure;
FIG. 2B is an illustration of a pressure sensor for use in a two-dimensional pressure sensor array according to the present disclosure;
FIG. 3 is a two-dimensional pressure sensor array according to the present disclosure;
FIG. 4A is a graph illustrating pressure distribution measured by a two-dimensional mechanical imaging system according to the present disclosure;
FIG. 4B illustrates a pressure distribution measured by a two-dimensional mechanical imaging system according to the present disclosure;
FIG. 5 is a graph illustrating a dynamic pressure distribution measured by a two-dimensional mechanical imaging system according to the present disclosure;
FIG. 6 is a graph illustrating a pressure distribution measured by a two-dimensional mechanical imaging system according to the present disclosure;
FIG. 7 is an image of a pressure distribution measured by the mechanical imaging system according to the present disclosure; and
FIG. 8 is flow chart of a method for mechanically imaging a specimen according to the present disclosure.

### DETAILED DESCRIPTION

A subject can perform a self-examination to check for cancer in their breast using, for example, palpation. Palpation involves the subject using their fingers to feel the anatomical texture of the breast and detect any variation therein. During palpation, a subject may detect a "lump," which may be indicative of breast cancer.

The present inventors determined that incorporating mechanical imaging, as a proxy for palpation, into routine mammography examination can enhance detection of breast cancer during a mammography visit. In particular, mechanical imaging may benefit subjects with high breast density where the breast cancer detection rate of X-ray imaging is reduced.

The present disclosure provides a two-dimensional mechanical imaging system and methods of use thereof. The system comprises a first substrate, a second substrate, an X-ray imaging system, and a two-dimensional pressure sensor array. The second substrate is spaced a first distance from the first substrate. The first substrate and the second substrate are positioned in an imaging field and are capable to move relative to one another during a compression cycle. The X-ray imaging system is capable to image the specimen in the imaging field. The two-dimensional pressure sensor array is positioned on the first substrate, the second substrate, or both the first substrate and the second substrate. The two-dimensional pressures sensor array is capable to measure a pressure distribution of the specimen during the compression cycle.

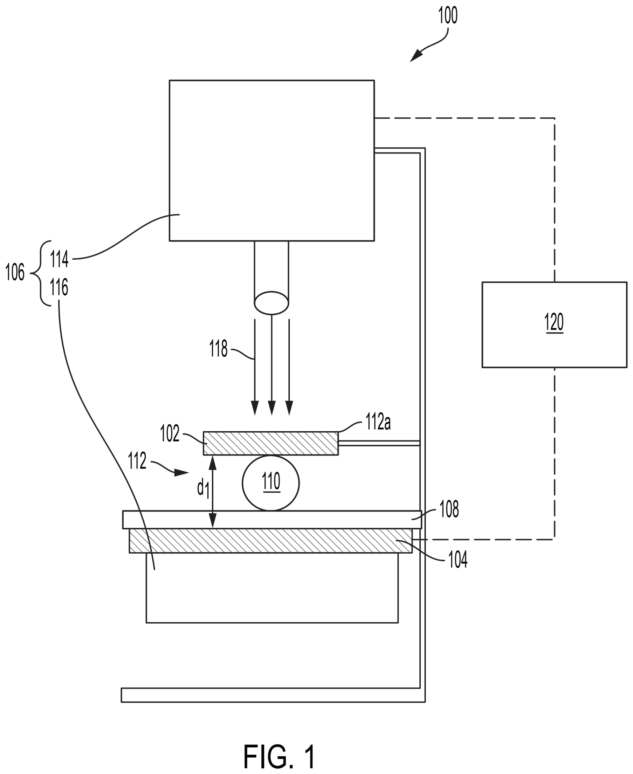

FIG. 1 illustrates a non-limiting embodiment of a two-dimensional mechanical imaging system 100 in accordance with the present disclosure. The system 100 comprises a first substrate 102, a second substrate 104, an X-ray imaging system 106, and a two-dimensional pressure sensor array 108. The X-ray imaging system 106 can define an imaging field 112 and a specimen 110 can be placed in the imaging field 112 intermediate the first and second substrates 102 and 104 for analysis. The system 100 can perform a compression cycle by moving the first and second substrates 102 and 104 relative to one another and compressing the portion of the specimen 110. The system 100 can X-ray image the specimen 110 using the X-ray imaging system 106 and measure a pressure distribution of the specimen 110 using the two-dimensional pressure sensor array 108 prior to, during, and/or after compression of the specimen 110. The X-ray imaging and the pressure distribution measurement can occur sequentially, at least partially overlap, or simultaneously.

Referring again to FIG. 1, the first and second substrates 102 and 104 are capable to be positioned in the imaging field 112 of the X-ray imaging system 106 and can be spaced a distance d₁ apart from each other. The first and second substrates 102 and 104 are capable of moving relative to one another to change the distance d₁.

In various embodiments, one of or both of the first and second substrates 102, 104 may move during a compression cycle. For example, first substrate 102 may move towards second substrate 104, second substrate 104 may move towards first substrate 102, or both the first and second substrates 102, 104 may move toward one another. The portion of the specimen 110 positioned in the imaging field 112, intermediate first substrate 102 and second substrate 104, can be subject to compression and decompression as a result of the movement of the first substrate 102 and/or second substrate 104.

During a compression cycle, the first distance d₁ can change between a first distance value and a second distance value. For example, the compression cycle can comprise a compression phase where the distance, d₁, between the first and second substrates 102 and 104 decreases from the first distance value towards the second distance value. The compression cycle can comprise a decompression phase, where the distance d₁ between the first and second substrates 102 and 104 increases from the second distance value towards the first distance value, thereby releasing compressive force applied during the compression phase. The compression cycle may include pauses at values intermediate the first distance value and the second distance value. In various non-limiting embodiments, the compression cycle can comprise compression phases and/or decompression phases that span values intermediate the first distance value and the second distance value.

The compression cycle can be configured to produce variable compressive forces depending on the portion of the specimen 110 to be imaged, the imaging/examination application, and/or the constraints of the system 100. During the compression phase of the compression cycle, the portion of the specimen 110 may be compressed to achieve a substantially uniform thickness of the specimen 110 across the first and second substrates 102 and 104, which can reduce overlap across the specimen 110 to enhance image quality. For example, in embodiments comprising mammographic exams, a range of 1 kPa to 20 kPa or 5 kPa to 15 kPa of compressive force may be exerted on the specimen 110 during the compression phase of the compression cycle to obtain a diagnostic quality image.

The first and second substrates 102 and 104 are positioned in the imaging field 112 of the X-ray imaging system 106 and may not substantially interfere with the operation of the X-ray imaging system 106. For example, the first and second substrates 102 and 104 can comprise a material capable of supporting the specimen 110 and can be substantially X-ray transparent. For example, the first and second substrates 102 and 104 can comprise a substantially X-ray transparent material, such as, for example, a polymeric material (e.g., an acrylic polymer) and/or a composite material (e.g., carbon fiber).

Referring again to FIG. 1, the two-dimensional pressure sensor array 108 can be positioned on first substrate 102, on second substrate 104, or on both first and second substrates 102, 104. The two-dimensional pressure sensor array 108 can be removably secured to the first substrate 102 and/or the second substrate 104 or permanently secured to the first substrate 102 and/or second substrate 104. For example, the two-dimensional pressure sensor array 108 can be removably secured to the first substrate 102 and/or second substrate 104 by various means capable of inhibiting the two-dimensional pressure sensor array 108 from substantially moving during the compression cycle, such as, for example, by a fastener (e.g., pin, hook and loop, screw, a snap), a strap, an adhesive, or any combination thereof. The two-dimensional pressure sensor array 108 may be removable, such that the system 100 may be used without the two-dimensional pressure sensor array 108 being within the imaging field 112 and/or the two-dimensional pressure sensor array 108 can be repositioned. For example, the system 100 may be used only with X-ray imaging system 106. With reference to FIG. 1, the two-dimensional pressure sensor array is shown on first substrate 104, but it will be understood that the two-dimensional pressure sensor array 108 alternatively may be positioned on second substrate 104 instead of the first substrate 102 as the application may require. In various non-limiting embodiments, a two-dimensional pressure sensor array may be positioned on both the first substrate 104 and the second substrate 104.

In various non-limiting embodiments, the two-dimensional pressure sensor array 108 may be built into the X-ray imaging system 106. For example, the two-dimensional pressure sensor array 108 may be built into a surface of the first substrate 102, the second substrate 104, the X-ray detector 116 of the X-ray imaging system 106, or a combination thereof.

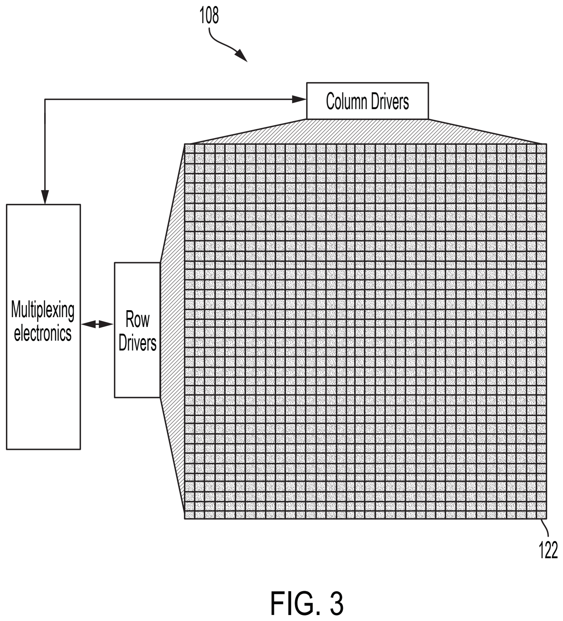

A detailed view of an embodiment of the pressure sensor array 108 is shown in FIG. 3. As illustrated, the two-dimensional pressure sensor array 108 can comprise a plurality of sensors 122 arranged in a two-dimensional array. Each sensor 122 can be capable of detecting a change in a capacitance, a resistance, or both the capacitance and resistance across the sensor 122. The change in capacitance, resistance, or both capacitance and resistance can be related to a pressure applied to the sensor 122.

In various non-limiting embodiments, the two-dimensional pressure sensor array 108 can comprise a pitch to allow for a certain number of sensors 122 in order to image the specimen at the required quality and resolution. In various non-limiting embodiments, the pitch of the two-dimensional pressure sensor array 108 can be at least 0.1 mm, such as, for example, at least 0.5 mm, at least 1 mm, at least 5 mm, at least 7 mm, or at least 10 mm. In various non-limiting embodiments, the pitch of the two-dimensional pressure sensor array 108 may be no greater than 20 mm such as, for example, no greater than 15 than mm, no greater than 10 than mm, no greater than 5 than mm, no greater than 3 than mm, or no greater than 1 mm. For example, the pitch of the two-dimensional pressure sensor array 108 can be in a range of 0.1 mm to 20 mm, such as, for example, 0.3 mm to 15 mm, 0.5 mm to 10 mm, or 1 mm to 5 mm.

As illustrated in FIG. 3, the two-dimensional pressure sensor array 108 can be substantially planar. The two-dimensional pressure sensor array 108 may be made by manufacturing multiple pressure sensors 122 on one or more silicon wafers. The silicon wafers may be used individually or may be tiled to create the two-dimensional pressure sensor array 108.

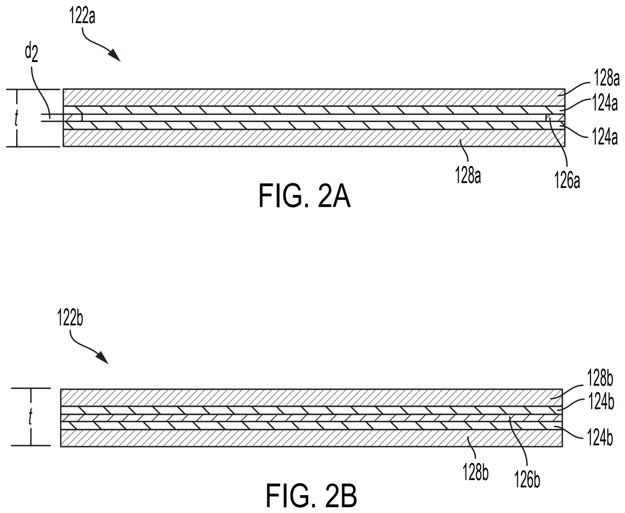

In various non-limiting embodiments, each pressure sensor 122 can be a solid-state pressure sensor, a pressure sensitive film, other pressure sensor configuration, or a combination thereof. For example, the pressure sensors 122 can comprise a solid-state pressure sensor 122a as shown in FIG. 2A. In certain embodiments, the solid-state pressure sensor 122a can include micro-electrical mechanical systems (MEMS) technology. As illustrated in FIG. 2A, the solid-state pressure sensor 122a may comprise at least two electrodes 124a, which may be separated by a spacer 126a. The solid-state pressure sensor 122a may further comprise a protective cover 128a surrounding the exterior of each electrode 124a to inhibit the specimen 110 from contacting the electrodes 124a. The solid-state pressure sensor 122a can determine pressure by measuring the capacitance and/or resistance between the two electrodes 124a as the electrodes 124a are deformed by the application of pressure to the pressure sensor 122a. The measured capacitance and/or resistance can be proportional to a distance d₂ between the electrodes 124a and, thus, indicative of the pressure applied to the two-dimensional pressure sensor array 108.

In various non-limiting embodiments, as illustrated in FIG. 2B, the sensors 122 can comprise a pressure sensor 122b comprising a pressure sensitive film 126b. The pressure sensitive film 126b can be positioned intermediate two electrodes 124b and encapsulated by the protective layer 128b. As pressure is applied across the pressure sensor 122b, the pressure sensitive film 126b may change electrical resistance and/or capacitance, which can be measured by the electrodes 124b. The measured change in electrical resistance and/or capacitance of the pressure sensitive film 126b can be indicative of the pressure applied to the two-dimensional pressure sensor array 108.

Referring again to FIG. 1, the two-dimensional pressure sensor array 108 can measure the pressure distribution of the specimen 110 positioned within the imaging field 112 of the X-ray imaging system 106. The pressure distribution can be used as a proxy for palpation, and the pressure distribution can be dynamically measured during a compression cycle.

The two-dimensional pressure sensor array 108 can be within the path of X-ray electromagnetic radiation 118 emitted from the X-ray imaging system 106 and passing through the specimen 110. During X-ray imaging, any material that is in the path of the X-ray electromagnetic radiation 118 can attenuate (e.g., absorb) X-ray electromagnetic radiation. It can be desirable to reduce attenuation of X-ray electromagnetic radiation for various reasons. For example, an undesirably high attenuation can interfere with the X-ray imaging by reducing image quality by either creating artifacts in the image and/or reducing the X-ray electromagnetic radiation 118 that can reach the X-ray detector 116.

The X-ray electromagnetic radiation 118 can be increased by increasing the output from the X-ray source 114 to overcome the attenuation. However, increasing the X-ray electromagnetic radiation output can increase the dose of X-ray electromagnetic radiation to the specimen 110. In the context of mammography examination, the United States Food and Drug Administration's established guidance as of 2024 provides that the X-ray electromagnetic radiation dosing to patients should be limited to levels "as low as reasonably achievable" because mammography examinations are a repeated screening modality and the dose delivered during mammography examinations is comparable to the maximum allowable annual X-ray electromagnetic radiation dose. Thus, reducing attenuation can reduce the amount of X-ray electromagnetic radiation that is dosed to the specimen 110.

Traditional mammography systems and mechanical imaging systems heretofore have not been combined into a single system capable of operating simultaneously for routine clinical use and mass deployment in breast cancer screening programs. The system 100 can balance X-ray electromagnetic radiation attenuation with the quality of X-ray and mechanical imaging.

The two-dimensional pressure sensor array 108 can be substantially X-ray transparent, which may reduce X-ray electromagnetic radiation attenuation caused by the two-dimensional pressure sensor array 108 as it may be positioned in the imaging field 112. For example, the two-dimensional pressure sensor array 108 can be constructed of materials which are substantially X-ray transparent or which otherwise cause minimal X-ray attenuation. For example, referring to FIGs. 2A and 2B, in embodiments comprising pressure sensor 122a and/or 122b, the electrodes 124a and/or 124b can comprise organic materials that are electrically conductive and X-ray transparent to reduce the impact of the two-dimensional pressure sensor array 108 on the X-ray image quality and/or the X-ray dose received by the specimen 110.

Referring back to FIG. 1, the two-dimensional pressure sensor array 108 may substantially comprise non-metallic materials such as, for example, a polymeric material (e.g., an organic material and/or a plastic). In various non-limiting embodiments, the non-metallic material can consist or consist essentially of a polymeric material. In various non-limiting embodiments, the two-dimensional sensor array can comprise metallic portions that are substantially positioned outside the imaging field 112, which may reduce X-ray attenuation caused by the two-dimensional pressure sensor array 108.

In various non-limiting embodiments, the two-dimensional pressure sensor array 108 can comprise a limited amount of metal in quantities which are able to be accounted for via calibration and which do not increase the X-ray electromagnetic radiation dose experienced by the subject to levels above regulatory limits. In various non-limiting embodiments, the thickness of any metallic component in the two-dimensional pressure sensor array 108 may be no greater than 10 µm such as, for example, no greater than 7 than µm, no greater than 5 µm, no greater than 3 µm, or no greater than 1 µm.

In various non-limiting embodiments, the two-dimensional pressure sensor array 108 can comprise a thickness, t, that may be adjusted to reduce attenuation of the X-ray electromagnetic radiation 118. In various non-limiting embodiments, the thickness, t, of the two-dimensional pressure sensor array 108 can be at least 1 µm, such as, for example, at least 3 µm, at least 5 µm, or at least 7 µm. In various non-limiting embodiments, the thickness, t, of the two-dimensional pressure sensor array 108 may be no greater than 50 µm such as, for example, no greater than 40 than µm, no greater than 30 µm, no greater than 20 µm, no greater than 10 µm, or no greater than 5 µm. For example, the thickness, t, of the two-dimensional pressure sensor array 108 can be in a range of 1 µm to 50 µm, such as, for example, 3 µm to 40 µm, 5 µm to 20 µm, or 7 µm to 10 µm.

The attenuation of the two-dimensional pressure sensor array 108 and the positioning of the two-dimensional pressure sensor array 108 within the X-ray imaging field 112 can be configured so as to not substantially affect the X-ray electromagnetic radiation dose received by the specimen 110 while still permitting desirable X-ray imaging. For example, in the case of breast cancer screening, the X-ray attenuation of the two-dimensional pressure sensor array 108 may be chosen to ensure that the patient 110 is not substantially exposed to an amount of X-ray electromagnetic radiation 118 greater than typically used during a conventional X-ray mammogram. In various non-limiting embodiments, the attenuation of the X-ray signal from the X-ray imaging system 106 by the two-dimensional pressure sensor array 108 can be no greater than 15%, such as, for example, no greater than 10%, no greater than 5%, no greater than 1%, or no greater than 0.5%.

The size and/or shape of the two-dimensional pressure sensor array 108 can be selected based on, for example, the portion of the specimen 110 to be imaged and/or measured, the size of the first substrate 102 and/or second substrate 104, the size of the X-ray detector 116, the size of the specimen 110, or any combination thereof.

Referring again to FIG. 1, the X-ray imaging system 106 can comprise an X-ray source 114 and an X-ray detector 116. The X-ray source 114 can be positioned on a first side 112a of the imaging field 112. The X-ray source 114 can be capable of emitting X-ray electromagnetic radiation 118 into the imaging field 112, such that the X-ray electromagnetic radiation 118 can pass through the specimen 110 positioned therein. The X-ray electromagnetic radiation 118 emitted by the X-ray source 114 may include electromagnetic waves with wavelengths in a range of 0.01 to 10 nanometers.

The X-ray source 114 may comprise a vacuum chamber comprising an anode and a cathode. To generate the X-ray electromagnetic radiation 118, high current can be applied to the cathode to increase its temperature, thereby allowing electrons to be released therefrom toward the anode. When the electrons impact the anode, the electrons can suddenly stop, causing a loss of energy to be released in the form of X-ray photons. This results in a beam of X-ray electromagnetic radiation 118 directed towards the specimen 110.

The X-ray detector 116 can be positioned on a second side of the imaging field 112. The X-ray detector 116 can be opposing the X-ray source 114 in order to detect at least a portion of the X-ray electromagnetic radiation 118 transmitted from the X-ray source 114 and passing through the specimen 110 in the imaging field 112. The X-ray detector 116 can convert the received electromagnetic radiation 118 into an electrical signal that can be used to produce an X-ray image of the specimen 110. The X-ray detector 116 can be an indirect detector or a direct detector. For example, the X-ray detector 116 can be a CMOS sensor, a CCD sensor, a scintillator detector, or a combination thereof.

In various non-limiting embodiments, the specimen 110 can be tissue, such as, for example, breast tissue of a subject. In certain non-limiting embodiments where the specimen 110 is breast tissue, the X-ray imaging system 106 can be a mammography system. The breast tissue can be compressed by the first and second substrates 102 and 104 (e.g., a compression paddle on the mammography system) such that the breast tissue has a generally uniform thickness across the first and second substrates 102 and 104 and tissue overlap is minimized. During the compression phase of the compression cycle, the X-ray source 114 can emit X-ray electromagnetic radiation 118 to produce the X-ray image.

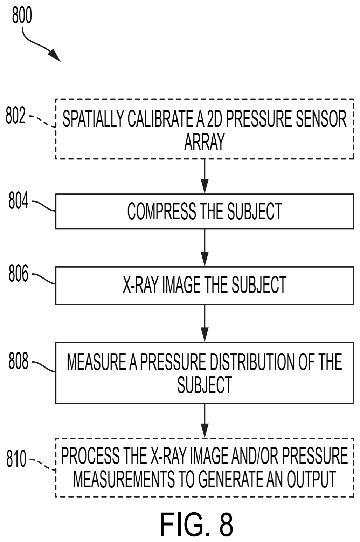

Referring to FIG. 8, a method for two-dimensional mechanical imaging of a specimen according to the present disclosure is provided. The method 800 can comprise compressing the specimen 100 positioned between the first substrate 102 and the second substrate 104 at step 804.

The method 800 can comprise obtaining an X-ray image of the specimen 110 at step 806 and measuring 808 a pressure distribution of the specimen 110 across the first substrate 102, the second substrate 104, or both the first and second substrates 102 and 104 at step 808. Steps 806 and 808 may occur sequentially, simultaneously, or at least partially overlap. Additionally, the order of steps 806 and 808 in FIG. 8 is for illustration purposes only and those steps may occur in any order that obtains desirable X-ray images and pressure distribution measurements.

Again referring to FIG. 8, the method 800 can optionally comprise processing the X-ray image and/or pressure measurements to generate an output at step 810. For example, the pressure distribution of the specimen measured at step 808 can be overlayed on the X-ray image of the specimen measured at step 806 to generate an overlayed image. In various non-limiting embodiments, a cartoon image or a physical outline of the specimen may be used in addition to or instead of the X-ray image. The overlayed image can be used to facilitate location of high-density regions of the specimen 110, which can enable a physician's examination of the breast tissue.

Referring again to FIG. 8, the method 800 can optionally comprise generating a dynamic pressure distribution map indicating a change in the pressure distribution during a compression cycle. For example, the system 100 can receive measurements from the two-dimensional pressure sensor array 108 and generate a dynamic pressure map, which shows pressure distribution as a function of time as well as space. The dynamic pressure map may be output as a stream (e.g., continuous, sequential, intermittent) of data at a rate that may depend on the desired application. The variation in the pressure distribution may be recorded over time throughout the compression cycle and dynamically mapped as a function of both time and space to illustrate additional features of the specimen 110. For example, in the context of breast tissue imaging, as the blood and fluid within the breast are compressed and decompressed, blood and fluid may flow out of and back into the breast. Observing and/or mapping such fluid flow may be indicative of clinically relevant anatomical features, such as, for example, a sclerotic mammary artery.

Prior to imaging the subject, the method 800 may optionally comprise spatially calibrating 802 the two-dimensional pressure sensor array 108 and the X-ray imaging system 106 relative to one another by registering data produced by the two-dimensional pressure sensor array 108 and data produced by the X-ray imaging system 106 to a shared coordinate system. Spatial calibration can ensure that the coordinate system of the X-ray imaging system 106 and the coordinate system of the two-dimensional pressure sensor 108 are aligned, such that the pressure distribution can be overlayed with the X-ray image of the specimen 110. Desired alignment of the coordinate systems of the two-dimensional pressure sensor array 108 and the X-ray imaging system 106 can enable artifacts in the imaging field, such as components of the two-dimensional pressure sensor array 108, to be removed (e.g., subtracted) from the X-ray image and/or overlayed image. For example, if the internal wiring of the two-dimensional pressure sensor array 108 attenuates a portion of the X-ray electromagnetic radiation, the internal sensor may appear in the X-ray image and/or overlayed image. Because the location of the components of the two-dimensional pressure sensor array 108 can be known relative to the two-dimensional pressure sensor array's coordinate system, spatial calibration of the two-dimensional pressure sensor array 108 relative to the X-ray imaging system 106 can allow the two-dimensional pressure sensor array 108 to be located and removed from the X-ray image based on the X-ray imaging systems 106 coordinate system.

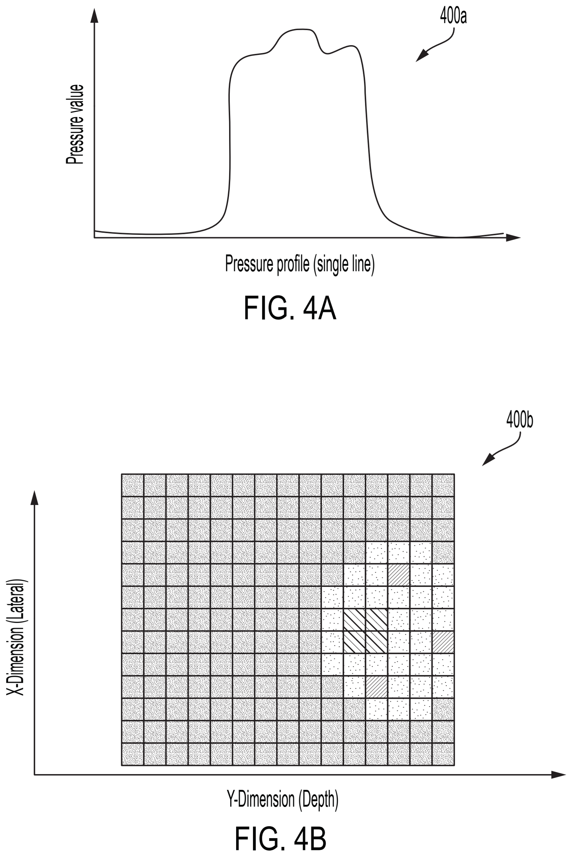

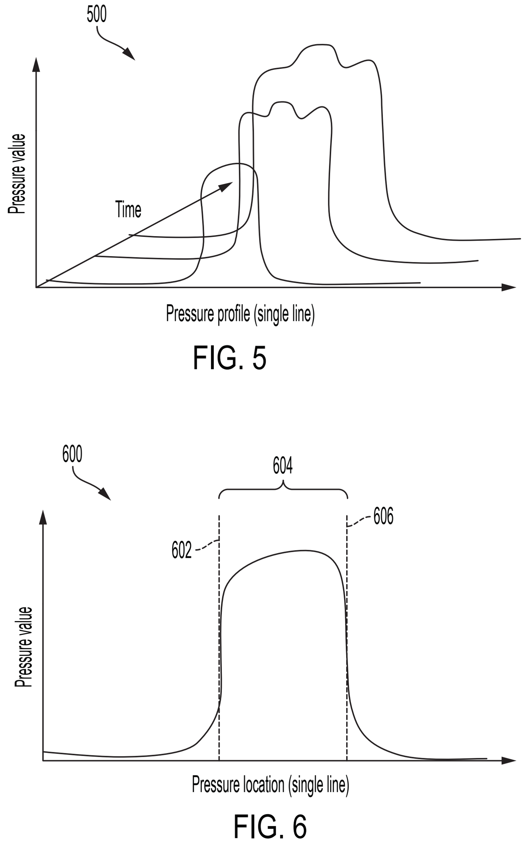

The two-dimensional pressure sensor array 108 can statically or dynamically measure pressure distributions of the specimen 110. For example, as illustrated in FIGs. 4A and 4B, the two-dimensional pressure sensor array 108 can be capable of measuring a pressure distribution 400a, 400b of the specimen 110 across the two-dimensional pressure sensor array 108 at a single point in time, thereby producing a static pressure distribution measurement. The two-dimensional pressure sensor array 108 can be capable of measuring at least two pressure distributions during at least two different states of compression of the subject, such as, for example, during different periods of the compression cycle. Such dynamic pressure measurements may produce a dynamic pressure map indicative of pressure distributions of the specimen 110 at multiple points in time throughout the compression cycle. A graph of the dynamic pressure distribution across a single line of the two-dimensional pressure sensor array is illustrated in FIG. 5.

The pressure distribution map illustrated in FIG. 4B, may change visual indicia (e.g., color) over time to indicate how the pressure distribution changed over time during the compression cycle. As shown in FIG. 4B, the pressure distribution may be output in a two-dimensional pressure map 400b. The two-dimensional pressure map 400b may be color-coded to differentiate between clinically relevant pressure differences. In various non-limiting embodiments, the two-dimensional pressure sensor array 108 may comprise ink-filled microballoons to create the two-dimensional pressure map 400b.

During both the compression and decompression phases, the relative pressure across the specimen 110 can change at different rates depending on the internal composition of the subject. For example, in the case of breast tissue, the relative pressure in the breast tissue may vary at different rates throughout the compression cycle depending on the density and elasticity of the breast anatomy features. Producing a dynamic pressure map showing the distribution of these changes as compression and decompression occurs can provide a physician with additional clinical data that may augment the clinical effectivity of breast cancer detection as compared to a standard mammography examination.

As shown in FIG. 6, the two-dimensional pressure sensor array 108 can measure pressure distribution as a function of location across the two-dimensional pressure sensor array 108. As shown, the pressure can increase from a nominal pressure to a peak pressure value, defining an edge 602 of the specimen 110. The peak pressure value can be sustained across the surface 604 of the specimen 110, and then reduced back to a nominal pressure value at an edge 606 of the specimen 110. This location-based pressure distribution 600 can be indicative of the size of the specimen 110 and the location of the specimen 110 on the two-dimensional pressure sensor array 108. Location of the specimen 110 across the two-dimensional pressure sensor array 108 can be used to ensure proper alignment of the specimen 110 within the overall imaging system 100. In various contexts, the size estimates based on the location-based pressure distribution 600 may also be of clinical value.

Referring back to FIG. 8, the method 800 can optionally comprise comparing the measured pressure distribution of the subject to a previous pressure distribution of the subject and identifying a change in the pressure distribution of the subject based on the comparison. The change may be indicative of an abnormality in the subject. The previous pressure distribution may be stored in memory in the system 100, or in a computer in signal communication with the system 100.

The pressure distributions may be observed by a user or stored for future reference. For example, the pressure distributions may be observed directly by a clinician or physician for diagnostic purposes and viewed over time to detect changes. In various non-limiting embodiments, the pressure distributions may be processed using artificial intelligence to identify, for example, an anatomical structure of the specimen.

Referring again to FIG. 1, imaging system 100 may further comprise a control circuit 120 in signal communication with first substrate 102, second substrate 104, the X-ray imaging system 106, and the two-dimensional pressure sensor array 108. The control circuit 120 can perform the method 800 as described with respect to FIG. 8.

As used herein, the term "control circuit" may refer to, for example, hardwired circuitry, programmable circuitry (e.g., a computer processor comprising one or more individual instruction processing cores, processing unit, processor, microcontroller, microcontroller unit, controller, digital signal processor (DSP), programmable logic device (PLD), programmable logic array (PLA), or FPGA), state machine circuitry, firmware that stores instructions executed by programmable circuitry, and any combination thereof. The control circuit 120 may, be embodied, collectively or individually, as circuitry that forms part of a larger system, for example, an IC, an ASIC, a SoC, a desktop computer, a laptop computer, a tablet computer, a server, a smart phone, etc. Accordingly, as used herein, a "control circuit" can comprise electrical circuitry having at least one discrete electrical circuit, electrical circuitry having at least one IC, electrical circuitry having at least one application-specific IC, electrical circuitry forming a general-purpose computing device configured by a computer program (e.g., a general-purpose computer configured by a computer program that at least partially carries out processes and/or devices described herein or a microprocessor configured by a computer program that at least partially carries out processes and/or devices described herein), electrical circuitry forming a memory device (e.g., forms of RAM), and/or electrical circuitry forming a communications device (e.g., a modem, communications switch, or optical-electrical equipment). The subject matter described herein may be implemented in an analog or digital fashion, or some combination thereof.

The control circuit 120 may be connected to a remote server, such as a cloud computing system. In various examples, the control circuit 120 may be located remotely or, alternatively, the control circuit 120 may be physically integrated in the imaging system 100. The control circuit 120 may include a mobile application for use on a mobile computing device, such as a smartphone or a tablet computer, for example. The control circuit 120 may be integrated into a hospital database, such that the resulting pressure distributions and any resulting diagnostics may be shared across a hospital database/platform.

In various non-limiting embodiments, the control circuit 120 may be capable of identifying an anatomical feature of the specimen 110 based on the pressure distributions measured by the two-dimensional pressure sensor array 108. Local increases in pressure exceeding an allowable deviation above or below the average pressure distribution of the specimen 110 may indicate a density change in the specimen 110. A density change in the specimen 110 may be indicative of an anatomical feature therein. For example, as illustrated in FIG. 4A and 4B, in the context of a mammography system for breast cancer screening, the control circuit 120 can be capable of detecting a lesion located within the breast. The breast contour may increase the pressure measured by the two-dimensional pressure sensor array 108. A lesion may have a higher density than average breast tissue, resulting in a higher-pressure measurement on the two-dimensional pressure sensor array 108. Based on the pressure distributions and/or the identification of the lesions, the control circuit 120 may trigger a flag indicating that a specific area should be examined more closely or refer the patient for additional diagnostic procedures.

In various non-limiting embodiments, the control circuit 120 may be capable of determining the stiffness of the specimen 110 in the imaging field 112 based on the measured pressure distribution. The control circuit 120 may be capable of estimating the density and/or elasticity of the subject110 based on the dynamic pressure distribution measured during the compression cycle, the compressive force applied during the compression cycle, the area over which the pressure distribution is measured, or any combination thereof.

For example, in the context of breast cancer screening, the control circuit 120 may be able to determine a breast density and/or elasticity of the breast based on the measured pressure distribution, the compressive force applied to the breast, and the size of the breast. Breast density may impact the effectivity of X-ray mammography, such that the effectivity of X-ray mammography may be reduced for patients with denser breasts. Current clinical practice, as mandated by the FDA, can include informing a patient of their breast density (e.g., "dense" or "not dense") at the time of the mammographic exam to determine whether additional imaging methods may be required or recommended. As such, standard mammography examination may be improved by outputting a breast density estimate determined by the control circuit 120 based, at least in part, on the measured pressure distribution.

In various non-limiting embodiments, the control circuit 120 may be able to analyze various data inputs, such as the pressure distribution, the size of the specimen 110, the history of the specimen 110, or any combination thereof, to determine an appropriate compressive force to be applied to the specimen 110 during the compression phase of the compression cycle. For example, the force required to drive the first and second substrates 102, 104 toward and away from each other during the compression cycle may be standardized between equipment manufacturers.

However, the actual pressure applied to the specimen 110 may vary depending on the surface area of the specimen 110 over which the compressive force is applied. By measuring the actual distribution of the pressure on the specimen 110, the maximum compressive force applied across the specimen 110 can be averaged to an optimal level. In various non-limiting embodiments, the optimal compressive force may be included in a feedback loop with the imaging system 100 to control the pressure applied to the specimen 110 and/or to stop compression when a measured applied pressure equals or exceeds the optimum applied pressure.

In various non-limiting embodiments, using the location-based pressure distribution 600 as illustrated in FIG. 6, the control circuit 120 may be capable of determining the position of the specimen 110 within the imaging field 112 based on the measured pressure distribution. Dynamic pressure distribution measurements may be used to detect errors in positioning of the specimen 110 during the compression cycle and allow for correction to improve comfort of the specimen 110 and/or reduce the risk of requiring re-imaging of the specimen 110. Improved positioning of the specimen 110 within the imaging field 112 can also improve the quality of the resulting X-ray image and/or pressure distribution measurements. For example, in the context of mammography examinations, accurate positioning of the breast relative to the imaging field 112 and, more specifically, the X-ray detector 116, can impact the diagnostic quality of the X-ray image, improve patient comfort, and reduce the risk of patient callback resulting from an unusable mammogram.

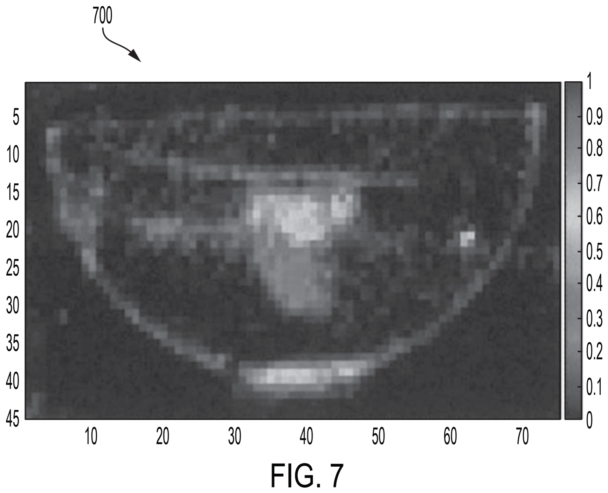

In various non-limiting embodiments, the control circuit 120 may be capable of displaying the pressure distribution to the user of the imaging system 100. The pressure distribution may take the form of any of the pressure distributions 400a, 400b, 500, 600, and/or 700. The control circuit 120 may be capable of outputting additional information based on the pressure distributions measured by the two-dimensional pressure sensor array 108. For example, the control circuit 120 can be capable to overlay the pressure distribution on an X-ray image of the specimen 110 in the imaging field 112 to generate an overlayed image 700 as illustrated in FIG. 7. As a result of the overlay, the final output may be an image of the specimen 110 showing the pressure distribution thereon.

The control circuit 120 may include and/or may be operably coupled to a user interface, which may be used by the user of the imaging system 100 to set up the two-dimensional pressure sensor array 108, calibrate the pressure measurements with respect to the X-ray imaging system 106, and initiate imaging by the user. The format of the pressure distribution may be selected by the user via a user interface and/or the pressure distributions may be output to the user via the user interface.

In various non-limiting embodiments, the control circuit 120 may be capable to transmit the pressure distribution, or any other estimate or determination based thereon, to a secondary location. The secondary location may be remote or may be operably connected to the control circuit. For example, the control circuit can be responsible for reporting various parameters to the X-ray imaging system, the two-dimensional pressure sensing array, the imaging system 100, the hospital database, or any combination thereof. The parameters being reported may include, but are not limited to, the pressure distribution measurement(s), pressure maps, density and/or elasticity of the subject, stiffness of the subject, reporting flags, etc.

The following numbered clauses are directed to various non-limiting embodiments according to the present disclosure:
Clause 1. An imaging system for imaging a tissue of a subject, the system comprising a first substrate; a second substrate spaced a first distance from the first substrate, wherein the first substrate and the second substrate are positioned in an imaging field, wherein the first substrate and the second substrate are capable to move relative to one another to during a compression cycle; an X-ray imaging system capable to image the specimen in the imaging field; and a two-dimensional pressure sensor array positioned on the first substrate, the second substrate, or both the first substrate and the second substrate, wherein the two-dimensional pressure sensor array is capable to measure a pressure distribution of the specimen during the compression cycle.
Clause 2. The system of Clause 1, wherein the two-dimensional pressure sensor array is removably secured to the first substrate, the second substrate, or both the first substrate and the second substrate.
Clause 3. The system of Clause 1 or 2, wherein the two-dimensional pressure sensor array is substantially X-ray transparent.
Clause 4. The system of any of Clauses 1-3, wherein the two-dimensional pressure sensor array consists of at least one material selected from the group consisting of a non-metallic material, an organic material, and plastic.
Clause 5. The system of any of Clauses 1-4, wherein the two-dimensional pressure sensor array attenuates an X-ray signal from the imaging system by 1% or less.
Clause 6. The system of any of Clauses 1-5, wherein the X-ray imaging system comprises an X-ray source positioned on a first side of the imaging field, the X-ray source capable to emit X-ray electromagnetic radiation toward the imaging field; and an X-ray detector positioned on a second side of the imaging field, wherein the X-ray detector is capable to detect at least a portion of the X-ray electromagnetic radiation passing through the specimen in the imaging field.
Clause 7. The system of any of Clauses 1-6, wherein the compression cycle comprises changing the first distance between a first value and a second value.
Clause 8. The system of Clause 7, wherein the two-dimensional pressure sensor array is capable to measure at least two pressure distributions during different time periods of the compression cycle.
Clause 9. The system of any of Clauses 1-8, wherein the pressure distribution is a dynamic pressure distribution map indicating a change in a pressure distribution across the two-dimensional pressure sensor array during at least a portion of the compression cycle.
Clause 10. The system of any of Clauses 1-9, wherein the X-ray imaging system is a mammography system and wherein the specimen in the imaging field is breast tissue.
Clause 11. The system of any of Clauses 1-10, wherein the system further comprises a control circuit in signal communication with the first substrate, the second substrate, the X-ray imaging system, and the two-dimensional pressure sensory array, the control circuit capable to overlay the pressure distribution on an X-ray image of the specimen in the imaging field.
Clause 12. The system of Clause 11, wherein the control circuit is capable to determine a stiffness of the specimen in the imaging field based on the pressure distribution.
Clause 13. The system of Clause 11 or 12, wherein the control circuit is capable to determine the position of the specimen in the imaging field.
Clause 14. The system of any of Clauses 1-13, wherein the two-dimensional pressure sensor array comprises a plurality of sensors, each of the sensors capable to detect a change in a capacitance, a resistance, or both the capacitance and resistance across the sensor, wherein the change is indicative of a pressure across the sensor.
Clause 15. A method for imaging a specimen, the method comprising compressing the specimen positioned between a first substrate and a second substrate; X-ray imaging the specimen; and measuring a pressure distribution of the specimen across the first substrate, the second substrate, or both the first substrate and the second substrate.
Clause 16. The method of Clause 15, wherein the method further comprises overlaying the pressure distribution of the specimen on the X-ray image of the specimen.
Clause 17. The method of Clause 15 or 16, wherein measuring the pressure distribution of the specimen occurs during at least two different states of compression of the specimen.
Clause 18. The method of any of Clauses 15-17, wherein the method further comprises generating a dynamic pressure distribution map indicating a change in the pressure distribution during the compressing the specimen.
Clause 19. The method of any of Clauses 15-18, wherein the method further comprises spatially calibrating a two-dimensional pressure sensor array for measuring the pressure distribution of the specimen and an X-ray imaging system for X-ray imaging the specimen relative to one another by registering data produced by the two-dimensional pressure sensor array and data produced by the X-ray imaging system to a shared coordinate system.
Clause 20. The method of any of Clauses 15-19, the method further comprising comparing the measured pressure distribution of the specimen to a historic pressure distribution of the specimen; and identifying a change in the pressure distribution of the specimen based on the comparison, wherein the change is indicative of an abnormality in the specimen.

Having thus described several aspects and embodiments of the technology of this application, it is to be appreciated that various alterations, modifications, and improvements will readily occur to those of ordinary skill in the art. Such alterations, modifications, and improvements are intended to be within the scope of the technology described in the application. It is, therefore, to be understood that the foregoing embodiments are presented by way of example only and that, within the scope of the appended claims and equivalents thereto, inventive embodiments may be practiced otherwise than as specifically described. In addition, any combinations of two or more features, systems, articles, materials, and/or methods described herein, if such features, systems, articles, materials, and/or methods are not mutually inconsistent, are included within the scope of the present disclosure.

In certain embodiments, a processor may be a physical or virtual processor. In other embodiments, a virtual processor may be spread across one or more portions of one or more physical processors. In certain embodiments, one or more of the embodiments described herein may be embodied in hardware such as a Digital Signal Processor (DSP). In certain embodiments, one or more of the embodiments herein may be executed on a DSP. One or more of the embodiments herein may be programmed into a DSP. In some embodiments, a DSP may have one or more processors and one or more memories. In certain embodiments, a DSP may have one or more computer readable storages. In many embodiments, a DSP may be a custom designed ASIC chip. In other embodiments, one or more of the embodiments stored on a computer readable medium may be loaded into a processor and executed.

Also, as described, some aspects may be embodied as one or more methods. The acts performed as part of the method may be ordered in any suitable way. Accordingly, embodiments may be constructed in which acts are performed in an order different than illustrated, which may include performing some acts simultaneously, even though shown as sequential acts in illustrative embodiments.

The phrase "and/or", as used herein in the specification and in the claims, should be understood to mean "either or both" of the elements so conjoined, i.e., elements that are conjunctively present in some cases and disjunctively present in other cases.

As used herein in the specification and in the claims, the phrase "at least one", in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified.

In the claims, as well as in the specification above, all transitional phrases such as "comprising," "including," "carrying," "having," "containing," "involving," "holding," "composed of," and the like are to be understood to be open-ended, i.e., to mean including but not limited to. The transitional phrases "consisting of" and "consisting essentially of" shall be closed or semi-closed transitional phrases, respectively.

Where a range or list of values is provided, each intervening value between the upper and lower limits of that range or list of values is individually contemplated and is encompassed within the disclosure as if each value were specifically enumerated herein. In addition, smaller ranges between and including the upper and lower limits of a given range are contemplated and encompassed within the disclosure. The listing of exemplary values or ranges is not a disclaimer of other values or ranges between and including the upper and lower limits of a given range.

Embodiments disclosed herein may be embodied as a system, method, or computer program product. Accordingly, embodiments may take the form of an entirely hardware embodiment, an entirely software embodiment (including firmware, resident software, microcode, etc.), or an embodiment combining software and hardware aspects that may all generally be referred to herein as a "circuit", "module", or "system". Furthermore, embodiments may take the form of a computer program product embodied in one or more computer readable medium(s) having computer readable program code embodied thereon.

## Claims

1. An imaging system for imaging a tissue of a subject, the system comprising:
a first substrate;
a second substrate spaced a first distance from the first substrate, wherein the first substrate and the second substrate are positioned in an imaging field, wherein the first substrate and the second substrate are capable to move relative to one another to during a compression cycle;
an X-ray imaging system capable to image the specimen in the imaging field; and
a two-dimensional pressure sensor array positioned on the first substrate, the second substrate, or both the first substrate and the second substrate, wherein the two-dimensional pressure sensor array is capable to measure a pressure distribution of the specimen during the compression cycle.

2. The system of claim 1, wherein the two-dimensional pressure sensor array is removably secured to the first substrate, the second substrate, or both the first substrate and the second substrate.

3. The system of claim 1 or 2, wherein the two-dimensional pressure sensor array is substantially X-ray transparent.

4. The system of claim 3, wherein the two-dimensional pressure sensor array consists of at least one material selected from the group consisting of a non-metallic material, an organic material, and plastic; and/or
wherein the two-dimensional pressure sensor array attenuates an X-ray signal from the imaging system by 1% or less.

5. The system of any of claims 1-4, wherein the X-ray imaging system comprises:
an X-ray source positioned on a first side of the imaging field, the X-ray source capable to emit X-ray electromagnetic radiation toward the imaging field; and
an X-ray detector positioned on a second side of the imaging field, wherein the X-ray detector is capable to detect at least a portion of the X-ray electromagnetic radiation passing through the specimen in the imaging field.

6. The system of any of claims 1-5, wherein the compression cycle comprises changing the first distance between a first value and a second value, wherein optionally the two-dimensional pressure sensor array is capable to measure at least two pressure distributions during different time periods of the compression cycle.

7. The system of any of claims 1-6, wherein the pressure distribution is a dynamic pressure distribution map indicating a change in a pressure distribution across the two-dimensional pressure sensor array during at least a portion of the compression cycle.

8. The system of any of claims 1-7, wherein the X-ray imaging system is a mammography system and wherein the specimen in the imaging field is breast tissue.

9. The system of any of claims 1-8, wherein the system further comprises a control circuit in signal communication with the first substrate, the second substrate, the X-ray imaging system, and the two-dimensional pressure sensory array, the control circuit capable to overlay the pressure distribution on an X-ray image of the specimen in the imaging field,
wherein optionally:
the control circuit is capable to determine a stiffness of the specimen in the imaging field based on the pressure distribution; and/or
the control circuit is capable to determine the position of the specimen in the imaging field.

10. The system of any of claims 1-9, wherein the two-dimensional pressure sensor array comprises a plurality of sensors, each of the sensors capable to detect a change in a capacitance, a resistance, or both the capacitance and resistance across the sensor, wherein the change is indicative of a pressure across the sensor.

11. A method for imaging a specimen, the method comprising:
compressing the specimen positioned between a first substrate and a second substrate; X-ray imaging the specimen; and
measuring a pressure distribution of the specimen across the first substrate, the second substrate, or both the first substrate and the second substrate.

12. The method of claim 11, wherein the method further comprises overlaying the pressure distribution of the specimen on the X-ray image of the specimen.

13. The method of claim 11 or 12, wherein measuring the pressure distribution of the specimen occurs during at least two different states of compression of the specimen,
wherein optionally the method further comprises generating a dynamic pressure distribution map indicating a change in the pressure distribution during the compressing the specimen.

14. The method of any of claims 11-13, wherein the method further comprises spatially calibrating a two-dimensional pressure sensor array for measuring the pressure distribution of the specimen and an X-ray imaging system for X-ray imaging the specimen relative to one another by registering data produced by the two-dimensional pressure sensor array and data produced by the X-ray imaging system to a shared coordinate system.

15. The method of any of claims 11-14, the method further comprising:
comparing the measured pressure distribution of the specimen to a historic pressure distribution of the specimen; and
identifying a change in the pressure distribution of the specimen based on the comparison, wherein the change is indicative of an abnormality in the specimen.
